# EUROPÄISCHE PATENTANMELDUNG

(11) **EP 1 949 931 A2**
(43) Veröffentlichungstag der Anmeldung: **30.07.2008**
(21) Anmeldenummer: 08004679.0
(22) Anmeldetag: 14.08.2003
(51) Int. Cl.: A61M 16/14, A61M 11/00

(54) **Vorrichtung zur Beeinflussung von Gasflüssen**

(30) Priorität: 19.08.2002 DE 10238683
(62) Teilanmeldung aus: 03792320.8
(71) Anmelder: Rist, Max, 85276 Pfaffenhofen (DE)
(72) Erfinder: Rist, Max, 85276 Pfaffenhofen (DE)
(74) Vertreter: Liebl, Thomas

(57) **Zusammenfassung**

Die Erfindung betrifft eine Vorrichtung zur Beeinflussung von Gasflüssen. Erfindungsgemäß ist eine Vorrichtung zur Richtungslenkung von Gasflüssen bei Verneblern in Hin- und Rückatmungsbereichen eines Beatmungsgerätes mit einer Bypasseinrichtung eine Vorrichtung zur Verbringung von Gasflüssen bei Vernebiern in Hin- und Rückatmungsbereichen eines Beatmungsgerätes mit einem vom Beatmungsgerät aus zeitgesteuerten Ventil vorgesehen, sodass das Todraumvolumen nicht mit Aerosol versehen wird. Beide Vorrichtungen ermöglichen eine Applikation eines Aerosols nur in der Inspirationsphase, eine Reduktion der applizierten Medikamentenmenge durch eine Reduktion der Vernebelung des Todraums, somit der Nebenwirkungen der Medikamente und eine ausreichende Befeuchtung der Atemwege.

## Beschreibung

Bei pulmonal erkrankten Patienten werden zur Therapie häufig Aerosole appliziert. Besondere Schwierigkeiten stellen bei der Anwendung von Aerosolen die beatmeten Patienten dar. Es werden entweder druckluftabhängige Düsenvernebler oder kontinuierlich aerosolproduzierende Vernebler wie z.B. Ultaschallvernebler bzw. piezoelektische Vernebler eingesetzt. Die Düsenvernebler werden normalerweise in der gemeinsamen Endstrecke für In- und Exspiration der Beatmungsschläuche kurz vor dem Tubus platziert und über die Beatmungsgeräte gesteuert, so dass sie nur während der Inspiration aktiv sind. Allerdings ist der Vernebler bis zum Ende der Inspiration tätig, sodass im gesamten Schlauchsystem distal des Verneblers mit Aerosol angereichertes Gas steht, so auch das Todraumvolumen mit Aerosol versehen wird. So kann zwar zwischen dem In- bzw. Exspirationsschenkel und Tubus der Regel ein Bakterienfilter platziert werden, der seinen Sinn auch in der Befeuchtung der Atemwege hat. Die starke Befeuchtung der ungenützten Gassäule (Todraumvolumen) vor allem mit großen Aerosolpartikeln aber bewirkt, dass sich der Filter mit Flüssigkeit voll saugt und damit einerseits an antibakterieller Wirkung verliert und andererseits der Widerstand des Filters so groß wird, dass er sich verschließt.

Die kontinuierlichen Vernebler wie z.B. Ultraschallvernebler bzw. piezoelektischen Vernebler stellen kleinere Partikel (durchschnittlichen Größe unter 7□m) her, die besser an den Einsatzort gelangen, verbunden mit geringeren Nebenwirkungen und geringeren Medikamentenkosten. Allerdings können diese Geräte nur im Inspirationsschenkel eines Beatmungsgerätes platziert werden, da sie kontinuierlich Aerosol produzieren und so in der gemeinsamen Endstrecke sowohl in der Inspiration als auch in der Exspiration Aerosol zum bzw. vom Patienten weg transportiert würde. Ein Filter würde sich so voll saugen und an Wirksamkeit verlieren, sich bzw. verschließen. Durch den Verzicht auf Filter erfolgt keine ausreichende keine Atemluftbefeuchtung, also miss ein Atemluftbefeuchter verwendet werden, der neben den Kosten auch ein hygienisches Problem (nosokomiale Pneumonie) darstellt.

Der Erfindung liegt das Problem zugrunde eine Vorrichtung in Verbindung mit Aerosol produzierenden Verneblern zu schaffen, mit der die oben beschriebenen Probleme vermieden werden können.

Dieses Problem wird durch die im Patentanspruch 1 (Formulierungsvorschlag) ausgeführten Merkmale, mit Hilfe der Vorrichtung zur Richtungslenkung von Gasflüssen bei Verneblern in Hin- und Rückatmungsbereichen eines Beatmungsgerätes, wobei eine Bypasseinrichtung vorgesehen ist, die beim Fluss des Gases in die eine Richtung das Aerosol mitnimmt, beim Fluss in die andere Richtung über einen Bypass am Vernebler vorbeigeführt, gelöst.

Das Problem lässt sich auch durch die im Patentanspruch 2 (Formulierungsvorschlag) ausgeführten Merkmale mit Hilfe der Vorrichtung zur Verbringung von Gasflüssen bei Verneblern in Hin- und Rückatmungsbereichen eines Beatmungsgerätes durch ein vom Beatmungsgerät aus zeitgesteuerten Ventil lösen, das sich vor dem Ende der Inspiration abhängig vom Atemzugvolumen, der Inspirationdauer und dem Inspirationdruck schließt, um das Todraumvolumen nicht mit Aerosol zu versehen.

Meine Erfindung ermöglicht die Platzierung von Verneblern (jeder Vernebler ist verwendbar) zwischen Filter und Tubus ohne Atemluftbefeuchter an der gemeinsamen Endstrecke mit dem Vorteil des geringeren Verbrauchs an Medikamenten, weniger Nebenwirkungen durch die applizierten Medikamente und der längeren Haltbarkeit der Filter.

Dies geschieht,
1. indem, gemäß Patentanspruch 1, über Ventile die Atemluft bei der Inspiration über den Vernebler geführt wird, bei der Exspiration durch sich schließende Ventile (passiv oder aktiv gesteuert) die Ausatemluft über einem Bypass vorbei am Vernebler geleitet wird. (Abb. 3). Um dies zu ermöglichen können die Ventile an verschiedenen Stellen in dieser Gerätschaft platziert werden. Auch besteht in dieser Vorrichtung die Möglichkeit mit Zusatzeinrichtungen (z.B. durch Ventilatoren) die Verteilung des Aerosols zu optimieren.
2. indem, gemäß Patentanspruch 2, sich über ein Steuerungskabel oder - schlauch ein Ventil während der Inspiration abhängig vom Atemzugvolumen, der Inspirationdauer und dem Inspirationdruck öffnet, sodass das Todraumvolumen nicht mit Aerosol versehen wird. Auch besteht in dieser Vorrichtung die Möglichkeit mit Zusatzeinrichtungen (z.B. durch Ventilatoren) die Verteilung des Aerosols zu optimieren.

Die Erfindung wird nachfolgend anhand einer Zeichnung näher erläutert.

Es zeigen

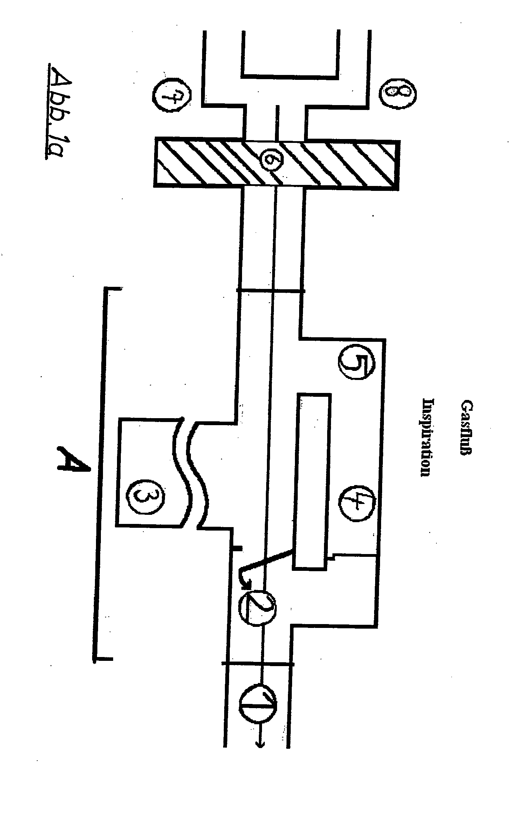

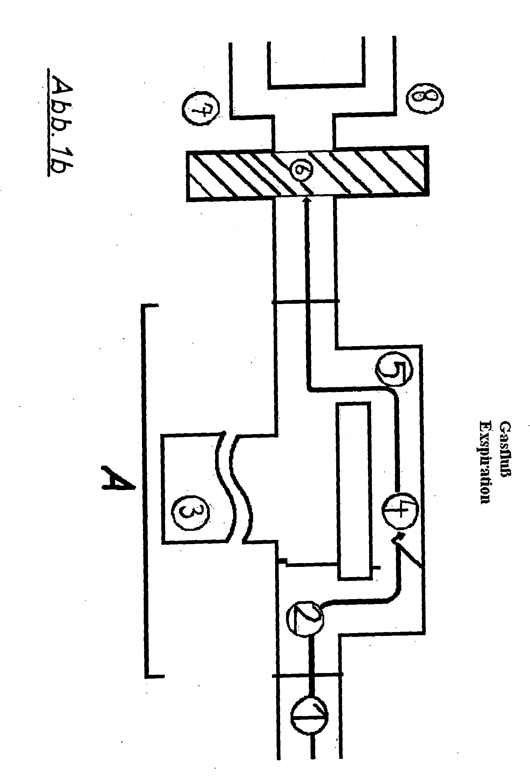

die Abbildungen 1 meine Erfindung gemäß Erfinderanspruch 1 (A). Sie ermöglicht eine Platzierung von Verneblern (jeder Vernebler ist verwendbar) (3) zwischen Filter (6) und Verbindungsstück zum Tubus (7) ohne

Atemluftbefeuchter an der gemeinsamen Endstrecke (1). Dies geschieht indem über Ventile (2, 4) (es können sämtliche möglichen Ventilarten sein) die Atemluft bei der Inspiration über den Vernebler geführt wird, indem sich das Ventil 2 öffnet und das Ventil 4 schließt (passiv oder aktiv gesteuert) (Abb. 1 a). Bei der Exspiration schließt sich Ventil 2 und öffnet sich Ventil 4 (passiv oder aktiv gesteuert), sodass die Ausatemluft über einem Bypass (5) am Vernebler vorbei geleitet wird (Abb. 1 b). Um dies zu ermöglichen können die Ventile an verschiedenen Stellen in dieser Gerätschaft platziert werden (Inspirationsventil: Zwischen Ein- und Auslass des Bypasses; Exspirationsventil: im ganzen Bypass). Der Inspirationsschenkel des Beatmungsschlauchs wird durch die Nummer 7 dargestellt, der Exspirationsschenkel durch die Nummer 8.

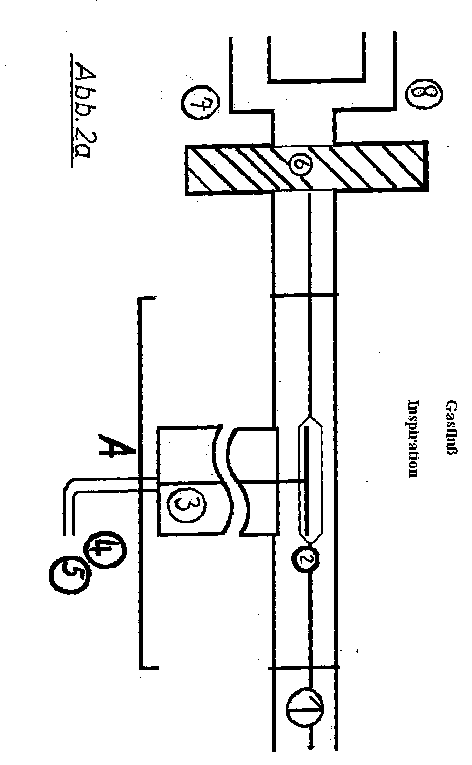

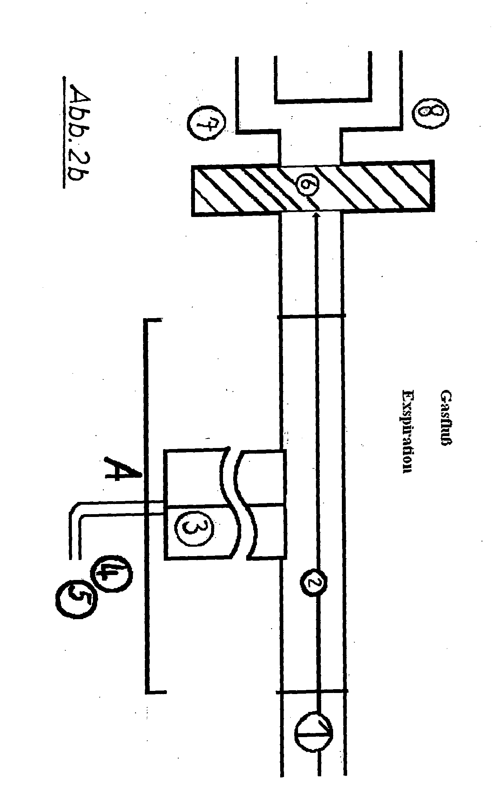

Die Abbildungen 2 zeigen eine Erfindung gemäß Erfinderanspruch 2 (A) (jeder Vernebler ist verwendbar), die über ein Steuerungskabel oder -schlauch (4), das Ventil (2) (es können sämtliche möglichen Ventilarten sein) während des in der Inspiration gewünschten Zeitraums abhängig vom Atemzugvolumen, der Inspirationdauer und dem Inspirationdruck gesteuert vom Beatmungsgerät (5) öffnet (Abb. 2a). In der endinspiratorischen Phase und in der Exspiration schließt das Ventil, sodass das Todraumvolumen nicht mit Aerosol versehen wird (Abb. 2b). Die Nummer 6 stellt den Filter dar. Der Inspirationsschenkel des Beatmungsschlauchs wird durch die Nummer 7 dargestellt, der Exspirationsschenkel durch die Nummer 8.

## Patentansprüche

1. Vorrichtung zur Richtungslenkung von Gasflüssen bei Aerosol produzierenden Verneblern in Hin- und Rückatmungsbereichen eines Beatmungsgerätes mit einer Bypasseinrichtung, die beim Fluss des Gases in die eine Richtung das Aerosol mitnimmt, beim Fluss in die andere Richtung über einen Bypass am Vernebler vorbeigeführt.

2. Vorrichtung zur Verbringung von Gasflüssen bei Verneblern in Hin- und Rückatmungsbereichen eines Beatmungsgerätes mit einem vom Beatmungsgerät aus zeitgesteuerten Ventil, sodass das Todraumvolumen nicht mit Aerosol versehen wird.
